# EUROPÄISCHE PATENTSCHRIFT

(11) **EP 1 056 500 B1**
(45) Veröffentlichungstag und Bekanntmachung des Hinweises auf die Patenterteilung: **17.11.2004**
(21) Anmeldenummer: 99907564.1
(22) Anmeldetag: 25.02.1999
(51) Int. Cl.: A61M 16/06

(54) **BEATMUNGSMASKE**
RESPIRATOR MASK
MASQUE RESPIRATOIRE

(30) Priorität: 25.02.1998 DE 19807961
(43) Veröffentlichungstag der Anmeldung: 06.12.2000
(73) Patentinhaber: MAP Medizin-Technologie GmbH, 82152 Martinsried (DE)
(72) Erfinder: LANG, Bernd, Christoph, D-82131 Gauting (DE)
(74) Vertreter: VOSSIUS & PARTNER
(86) Internationale Anmeldenummer: PCT/EP1999/001218
(87) Internationale Veröffentlichungsnummer: WO 1999/043375

(56) Entgegenhaltungen:
- EP-A- 0 462 701
- WO-A-96/28207
- DE-A- 19 548 380
- FR-A- 522 485
- GB-A- 1 315 893
- US-A- 4 201 205

## Beschreibung

Die vorliegende Erfindung betrifft eine Beatmungsmaske, die an verschiedene Gesichtsformen mehrfach anformbar ist.

Herkömmliche Beatmungsmasken bestehen im allgemeinen aus einem Maskenteil, der über die Nase und/oder den Mund eines zu beatmenden Patienten gebracht wird, einem Beatmungsschlauch zur Bereitstellung von Beatmungsluft und/oder zum Abtransport von ausgeatmeter Luft und Befestigungsmitteln zum Befestigen der Beatmungsmaske am Patienten. Der Maskenteil selbst weist einen relativ flexiblen Randbereich, der durch einen entsprechenden Anpreßdruck an die Gesichtsform eines Patienten andrückbar ist, und einen im Vergleich zu diesem Randbereich relativ steifen Mittelbereich auf, an dem z.B. der Beatmungsschlauch sowie Befestigungsbänder befestigt sind.

Herkömmliche Beatmungsmasken dieser Art haben insbesondere den Nachteil, daß zum Erreichen einer guten Paßform und zum Abdichten des Randbereiches der Maske am Gesicht eines zu beatmenden Patienten ein relativ großer Anpreßdruck erforderlich ist, was beim Tragen der Maske beim Patienten unter Umständen zu Beschwerden, wie z.B. Kopfschmerzen, Druck oder Wundstellen, führen kann. Ein weiterer Nachteil der herkömmlichen Beatmungsmaske besteht darin, daß sie aus verschiedenen Materialien gefertigt werden muß, um einerseits den tragenden Mittelbereich und andererseits den flexiblen Randbereich der Maske bereitstellen zu können. Ferner ist es bei herkömmlichen Beatmungsmasken schwierig, die Maske an verschiedene Kopfgrößen anzupassen, weil durch den steifen Mittelbereich des Maskenteils bereits eine bestimmte Form und Größe des Nasen- und/oder Mundteils vorgegeben ist, so daß sich der Randbereich unter Umständen auch bei einem großen Anpreßdruck nicht mehr vollständig an das Gesicht des Patienten anlegt.

Die DE-A-195 48 380 betrifft eine Atemmaske mit einem im wesentlichen schalenartigen formstabilen Grundkörper, an dem ein Anschlußknie, eine Formlasche mit einem Distanzkissen und eine Anschlußmaske vorgesehen sind. Die Anschlußmaske ist der Teil der Atemmaske, der am Gesicht des Patienten zur Anlage kommt. Zumindest am Randbereich eines Durchbruchs für die Nase eines zu beatmenden Patienten ist die Anschlußmaske aus einem bei Temperatureinwirkung oberhalb der Raumtemperatur plastisch verformbaren und bei tieferer Temperatur starren Werkstoff gebildet.

Es ist die Aufgabe der vorliegenden Erfindung, eine verbesserte Beatmungsmaske zur Verfügung zu stellen. Diese Aufgabe wird mit den Merkmalen der Ansprüche gelöst.

Dabei geht die Erfindung von dem Grundgedanken aus, daß die über die Nase und/oder den Mund eines Patienten anlegbare Beatmungsmaske zumindest im Randbereich aus einem verformbaren Werkstoff besteht, der von einer Stützstruktur (Stützrahmen oder -element) getragen wird, die bei üblichen Umgebungstemperaturen steif ist, jedoch bei Temperaturerhöhungen plastisch verformbar ist. Dadurch kann die für die Anpassung an die Gesichtsform des Patienten relevante Form der Beatmungsmaske bei einer erhöhten Temperatur wiederholt verändert werden, um so die Beatmungsmaske jeweils an verschiedene Gesichtsformen der Patienten anpassen zu können.

Vorteilhaft an der erfindungsgemäßen Beatmungsmaske ist insbesondere, daß die Maske wiederholt plastisch verformbar ist, um sie an verschiedene Gesichtsformen anzupassen, daß der Tragekomfort erhöht wird, d.h. eine bessere Paßform bei gleichzeitig geringerem Anpreßdruck gewährleistet wird, daß die Maske wahlweise nur aus einem Material für den gesamten Maskengrundkörper oder aus verschiedenen Materialien, z.B. für den Mittelbereich und den Randbereich, bestehen kann und daß sie kostengünstig hergestellt und entsorgt werden kann.

Die Erfindung wird im folgenden anhand bevorzugter Ausführungsformen beispielhaft beschrieben. Die Zeichnungen zeigen in:
- Fig. 1: eine erste Ausführungsform der erfindungsgemäßen Beatmungsmaske am Patienten in Vorderansicht;
- Fig. 2: eine Seitenansicht von Fig. 1;
- Fig. 3: eine zweite Ausführungsform der erfindungsgemäßen Beatmungsmaske am Patienten in Vorderansicht; und
- Fig. 4: eine Seitenansicht von Fig. 3.

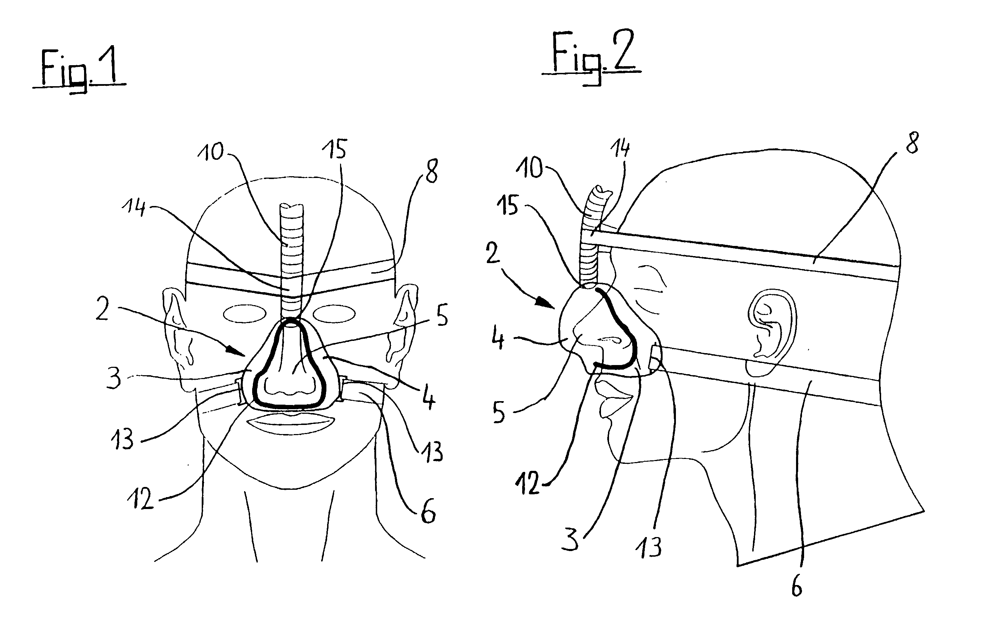

Die in den Figuren 1 und 2 dargestellte Beatmungsmaske 2 weist einen über die Nase und/oder den Mund eines Patienten anlegbaren Maskengrundkörper 4 auf, der durch Befestigungsmittel 6 und 8, z.B. elastische Bänder, am Kopf des Patienten befestigbar ist. Der Maskengrundkörper 4 besteht vorzugsweise aus einem gut verformbaren Randbereich 3 und einem im Vergleich zu diesem Randbereich 3 relativ steifen Mittelbereich 5. Der Maskengrundkörper 4 ist ferner, vorzugsweise im Mittelbereich 5, mit einem Beatmungsschlauch 10 verbunden, der mit einer Beatmungsvorrichtung (nicht dargestellt) in Verbindung steht, um dem Patienten Beatmungsluft zuzuführen und/oder um ausgeatmete Luft von ihm abzufahren.

Der Maskengrundkörper 4, insbesondere der Randbereich 3 der Beatmungsmaske 2 besteht aus einem hautverträglichen und leicht reinigbaren Material, vorzugsweise aus einem einzigen Material, insbesondere einem Kunststoff, wie z.B. Silikon oder Polyurethan, d.h. der Mittelbereich 5 und der Randbereich 3 bestehen vorzugsweise aus dem gleichen Material jedoch unterschiedlicher Festigkeit (Härte) und/oder Materialstärke.

Die Beatmungsmaske 2 weist ferner eine den Maskengrundkörper 4 bzw. den Randbereich 3 tragende Stützstruktur 12 auf, die innerhalb, außerhalb oder im Maskengrundkörper 4 angeordnet sein kann. Die Stützstruktur 12 ist vorzugsweise, wie in Figuren 1 und 2 dargestellt, in Form eines Stützrahmens im Randbereich 3 der Beatmungsmaske 2 gebildet, kann jedoch auch in Form mehrerer Stützelemente oder einer Kombination von stützrahmen und Stützelementen ausgebildet sein. Ferner ist es auch möglich anstelle des Stützrahmens oder zusätzlich dazu ein schalenförmig ausgebildetes, d.h. dreidimensionales Stützelement (ohne Abbildung) vorzugsweise im Bereich der oder um die Nase des Patienten vorzusehen. Dadurch wird die Stabilität der erfindungsgemäßen Beatmungsmaske, insbesondere die der Stützstruktur 12 weiter erhöht.

Die Stützstruktur 12 besteht vorzugsweise aus einem Material, das bei relativ niedrigen Temperaturen, z.B. 70°C, wiederholt plastisch verformbar und bei der Gebrauchstemperatur, z.B. unterhalb 40°C, relativ steif ist und in diesem Zustand die Stützfunktion erfüllt. Geeignete Materialien für die Stützstruktur sind z.B. Bi-, BiCd- sowie In-Legierungen, thermoverformbare Kunststoffe und/oder Wachse mit einem geeignet definierten Erweichungspunkt oder -bereich. So können z.B. durch geeignete Wahl der Zusammensetzung der Legierung, des Kunststoffes oder des Wachses die physikalischen Parameter, wie die Schmelztemperatur, der Erweichungsbereich und die mechanischen Eigenschaften, des Materials für die Stützstruktur 12 eingestellt werden.

Zur Anpassung der Beatmungsmaske 2 an die Gesichtsform eines Patienten wird der Maskengrundkörper 4 zusammen mit der Stützstruktur 12 bis mindestens zur zuvor erwähnten Grenztemperatur erwärmt, etwa durch Einlegen in geeignet erhitztes Wasserbad, so daß die Stützstruktur 12 ohne großen Kraftaufwand plastisch verformbar ist. Anschließend wird die Beatmungsmaske 2 vorzugsweise schnell abgekühlt, so daß der Maskengrundkörper 4 eine für den Patienten verträgliche Temperatur hat, während die Stützstruktur 12 weiterhin plastisch verformbar ist. Indem die Beatmungsmaske 2 nun am Gesicht des Patienten angelegt wird, kann die Stützstruktur 12 und der Randbereich 3 entsprechend der Gesichtsform angepaßt werden bzw. paßt sich automatisch an. Die Ummantelung der Stützstruktur 12 ist so ausgebildet, daß die beim Verfestigen frei werdende Wärme vorzugsweise vom Patienten weg geleitet wird, d.h. die Stützstruktur 12 liegt bevorzugt nahe der vom Patienten wegweisenden Oberfläche der Beatmungsmaske 2. Nachdem auch die Stützstruktur 12 abgekühlt ist, behält auch der Randbereich 3 der Beatmungsmaske 2 die individuell an die Gesichtsform des Patienten angepaßte Form. Dieser Vorgang kann beliebig oft für beliebige Gesichtsformen wiederholt werden.

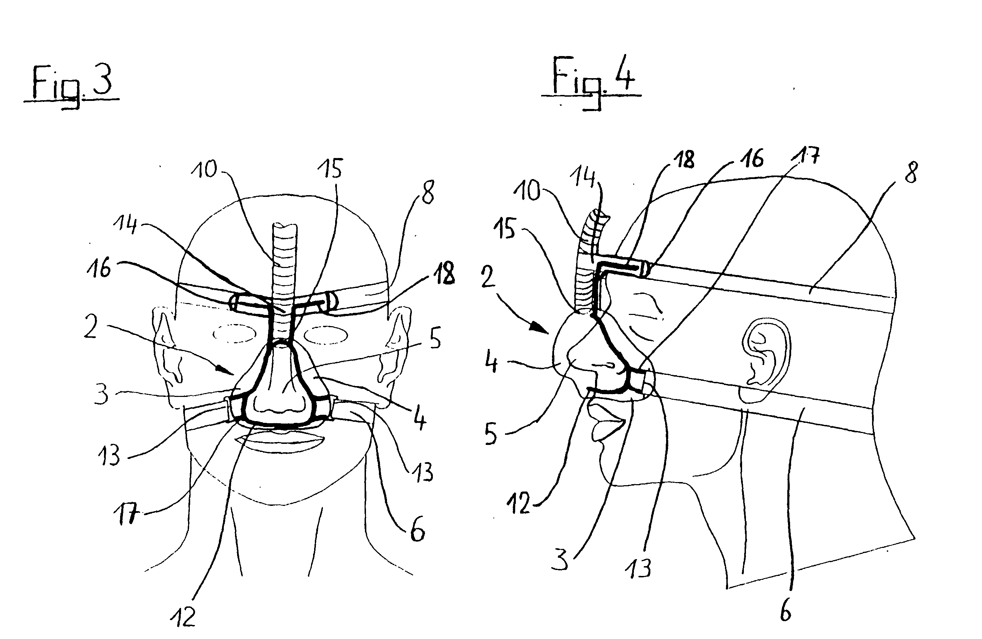

Eine zweite Ausführungsform der erfindungsgemäßen Beatmungsmaske 2 ist in den Figuren 3 und 4 gezeigt. Diese Ausführungsform entspricht im wesentlichen der vorstehend beschriebenen, wobei gleiche Elemente mit denselben Bezugszeichen versehen sind.

Die in den Figuren 3 und 4 dargestellte Beatmungsmaske 2 unterscheidet sich von der in Figuren 1 und 2 beschriebenen im wesentlichen dadurch, daß ferner Stützstrukturen 17 für Befestigungsstellen 13 der Befestigungsmittel 6 und 8 und/oder Stützstrukturen 18 für eine Maskenfixierungsstelle 14 und/oder eine Beatmungsschlauchanschlußstelle 15 im Maskengrundkörper 4 vorgesehen sind, die im wesentlichen die gleichen Eigenschaften aufweisen, wie die zuvor beschriebene Stützstruktur 12. Mit derartigen Stützstrukturen für die Befestigungseinrichtungen 13, 14 und/oder 15 kann die Paßform der Maske 2 weiter individuell für die Patienten verbessert werden.

Vorzugsweise kann sich dazu der Maskengrundkörper 4 bis zur Maskenfixierungsstelle 14 und/oder zumindest teilweise in Richtung der Befestigungsmittel 8 erstrecken. Ferner kann an der Stützstruktur 18 ebenfalls ein Grundkörper entsprechend dem Maskengrundkörper vorgesehen sein, der mit dem Maskengrundkörper 4 verbunden ist. In diesen Fällen kann es vorteilhaft sein, an dem sich im Bereich der Stützstruktur 18 erstrechkenden Abschnitt des Grundkörpers 4 Befestigungselemente 16 für das Befestigungsmittel 8 vorzusehen.

## Patentansprüche

1. Beatmungsmaske (2) mit einem Maskengrundkörper (4), der einen relativ flexiblen Randbereich aufweist und der an verschiedene Gesichtsformen flexibel anpaßbar ist, und mit einer den Maskengrundkörper (4) tragenden Stützstruktur (12, 17, 18), die oberhalb einer bestimmten Grenztemperatur wiederholt plastisch verformbar ist, um die Beatmungsmaske (2) an verschiedene Gesichtsformen wiederholt anzupassen.

2. Beatmungsmaske (2) nach Anspruch 1, wobei der Maskengrundkörper (4) aus einem verformbaren Randbereich (3) und einem im Vergleich dazu steifen Mittelbereich (5) besteht und die Stützstruktur (12) den Randbereich (3) trägt.

3. Beatmungsmaske (2) nach Anspruch 1 oder 2, wobei sie ferner Befestigungsmittel (6, 8) zur Befestigung der Maske am Patienten und einen Beatmungsschlauch (10) zur Bereitstellung von Beatmungsluft und/oder zum Abführen von ausgeatmeter Luft aufweist.

4. Beatmungsmaske (2) nach einem der vorhergehenden Ansprüche, wobei zumindest der Randbereich (3) des Maskengrundkörpers (4) aus einem wiederholt verformbaren, hautverträglichen und leicht reinigbaren Material besteht.

5. Beatmungsmaske (2) nach Anspruch 4, wobei das Material Silikon oder Polyurethan ist.

6. Beatmungsmaske (2) nach einem der vorhergehenden Ansprüche, wobei die Stützstruktur (12) aus einer Metallegierung, einem thermoverformbaren Kunststoff und/oder einem Wachs gebildet ist.

7. Beatmungsmaske (2) nach Anspruch 6, wobei die Metallegierung, eine Bi-, BiCd- oder In-Legierung ist.

8. Beatmungsmaske (2) nach einem der vorhergehenden Ansprüche, wobei die Grenztemperatur vorzugsweise zwischen 40°C und 120°C liegt.

9. Beatmungsmaske (2) nach einem der vorhergehenden Ansprüche, wobei die Stützstruktur (12) relativ nahe an einer vom Patienten wegweisenden Oberfläche des Maskengrundkörpers (4) liegt.

10. Beatmungsmaske (2) nach einem der Ansprüche 3 bis 9, wobei der Maskengrundkörper (4) mindestens eine weitere Stützstruktur (17) für Befestigungsstellen (13) der Befestigungsmittel (6, 8) und/oder mindestens eine weitere Stützstruktur (18) an einer Maskenfixierungsstelle (14) im Bereich der Stirn des Patienten und/oder an einer Beatmungsschlauchanschlußstelle (15) aufweist.

11. Beatmungsmaske (2) nach Anspruch 10, wobei die mindestens eine weitere Stützstruktur oberhalb einer bestimmten Temperatur wiederholt plastisch verformbar ist.

12. Beatmungsmaske (2) nach einem der Ansprüche 3 bis 10, wobei sich die Stützstruktur (12) in den Bereich einer Maskenfixierungsstelle (14) an der Stirn des Patienten und/oder den Bereich von Befestigungsstellen (13) der Befestigungsmittel (6, 8) erstreckt.

13. Beatmungsmaske (2) nach einem der vorhergehenden Ansprüche, wobei die Stützstruktur (12) schalenförmig ausgebildet ist.

14. Beatmungsmaske (2) nach Anspruch 13, wobei die schalenförmige Ausbildung im Bereich um die Nase des Patienten vorgesehen ist.

## Claims

1. A respirator mask (2) comprising a mask main body (4) which has a relatively flexible edge region and which can be flexibly adapted to different facial shapes, and a support structure (12, 17, 18) which carries the mask main body (4) and which is repeatedly plastically deformable above a given limit temperature in order to repeatedly adapt the respirator mask (2) to different facial shapes.

2. A respirator mask (2) according to claim 1 wherein the mask main body (4) comprises a deformable edge region (3) and a central region (5) which is stiff in comparison therewith, and the support structure (12) carries the edge region (3).

3. A respirator mask (2) according to claim 1 or claim 2 wherein it further has fixing means (6, 8) for fixing the mask to the patient and a respirator hose (10) for providing respiration air and/or for carrying away exhaled air.

4. A respirator mask (2) according to one of the preceding claims wherein at least the edge region (3) of the mask main body (4) comprises a repeatedly deformable, skin-compatible and easily cleanable material.

5. A respirator mask (2) according to claim 4 wherein the material is silicone or polyurethane.

6. A respirator mask (2) according to one of the preceding claims wherein the support structure (12) is formed from a metal alloy, a heat-deformable plastic material and/or a wax.

7. A respirator mask (2) according to claim 6 wherein the metal alloy is a Bi-, BiCd- or In-alloy.

8. A respirator mask (2) according to one of the preceding claims wherein the limit temperature is preferably between 40°C and 120°C.

9. A respirator mask (2) according to one of the preceding claims wherein the support structure (12) is relatively close to a surface, which faces away from the patient, of the mask main body (4).

10. A respirator mask (2) according to one of claims 3 to 9 wherein the mask main body (4) has at least one further support structure (17) for fixing locations (13) of the fixing means (6, 8) and/or at least one further support structure (18) at a mask securing location (14) in the region of the forehead of the patient and/or at a respirator hose connecting location (15).

11. A respirator mask (2) according to claim 10 wherein the at least one further support structure is repeatedly plastically deformable above a given temperature.

12. A respirator mask (2) according to one of claims 3 to 10 wherein the support structure (12) extends into the region of a mask securing location (14) at the forehead of the patient and/or the region of fixing locations (13) of the fixing means (6, 8).

13. A respirator mask (2) according to one of the preceding claims wherein the support structure (12) is of a shell-shaped configuration.

14. A respirator mask (2) according to claim 13 wherein the shell-shaped configuration is provided in the region around the nose of the patient.

## Revendications

1. Masque respiratoire (2) comportant un corps de base du masque (4) qui présente une zone de bord relativement souple qui est souplement adaptable à différentes formes de visage, et une structure d'appui (12, 17, 18) portant le corps de base du masque (4), qui au-dessus d'une température limite déterminée peut être plastiquement déformée de manière répétitive, pour réadapter le masque respiratoire (2) de manière répétitive à différentes formes de visage.

2. Masque respiratoire (2) selon la revendication 1, le corps de base du masque (4) consistant en une zone de bord (3) déformable et une zone médiane rigide (5) en comparaison et la structure d'appui (12) portant la zone de bord (3).

3. Masque respiratoire (2) selon les revendications 1 ou 2, présentant par ailleurs des moyens de fixation (6, 8) pour fixer le masque au patient et un tuyau respiratoire (10) pour l'amenée de l'air de respiration et/ou évacuer l'air expiré.

4. Masque respiratoire (2) selon l'une quelconque des revendications précédentes, dans lequel au moins la zone de bord (3) du corps de base du masque (4) consiste en un matériau pouvant être déformé de manière répétitive, bien toléré par la peau et facile à nettoyer.

5. Masque respiratoire (2) selon la revendication 4, le matériau étant de la silicone ou du polyuréthane.

6. Masque respiratoire (2) selon l'une quelconque des revendications précédentes, la structure d'appui (12) étant formée dans un alliage de métal, un plastique thermodéformable et/ou une cire.

7. Masque respiratoire (2) selon la revendication 6, l'alliage de métal étant un alliage de Bi, BiCd ou In.

8. Masque respiratoire (2) selon l'une quelconque des revendications précédentes, la température limite se situant de préférence entre 40°C et 120°C.

9. Masque respiratoire (2) selon l'une quelconque des revendications précédentes, la structure d'appui (12) se situant relativement proche d'une surface du corps de base du masque (4) dirigée en s'écartant du patient.

10. Masque respiratoire (2) selon l'une quelconque des revendications 3 à 9, le corps de base du masque (4) présentant au moins une structure d'appui (17) supplémentaire pour les points de fixation (13) des moyens de fixation (6, 8) et/ou au moins une structure d'appui (18) supplémentaire à l'un des points de fixation du masque (14) dans la zone du front du patient et/ou à un point de raccordement du tuyau respiratoire (15).

11. Masque respiratoire (2) selon la revendication 10, la structure d'appui supplémentaire qui est au moins au nombre de une pouvant être plastiquement déformée de manière répétitive au-dessus d'une température déterminée.

12. Masque respiratoire (2) selon l'une quelconque des revendications 3 à 10, la structure d'appui (12) s'étendant dans la zone d'un point de fixation du masque (14) au front du patient et/ou dans la zone de points de fixation (13) des moyens de fixation (6, 8).

13. Masque respiratoire (2) selon l'une quelconque des revendications précédentes, la structure d'appui (12) étant formée comme coque.

14. Masque respiratoire (2) selon la revendication 13, la configuration en forme de coque étant prévue dans la zone autour du nez du patient.
